# EUROPÄISCHE PATENTSCHRIFT

(11) **EP 3 615 169 B1**
(45) Veröffentlichungstag und Bekanntmachung des Hinweises auf die Patenterteilung: **24.08.2022**
(21) Anmeldenummer: 18721019.0
(22) Anmeldetag: 25.04.2018
(51) Int. Cl.: B01D 21/26, B01L 3/00, G01N 33/49, A61B 5/15

(54) **TRENNKÖRPER**
SEPARATING ELEMENT
CORPS DE SÉPARATION

(30) Priorität: 26.04.2017 DE 102017108935
(43) Veröffentlichungstag der Anmeldung: 04.03.2020
(73) Patentinhaber: Sarstedt Aktiengesellschaft & Co. KG, 51588 Nümbrecht (DE)
(72) Erfinder: WEINSTOCK, Mark, 57612 Helmenzen (DE); WEGENER, Christian, 51588 Nümbrect (DE); KARRENBERG, Ulrich, 51709 Marienheide (DE)
(74) Vertreter: Grosse, Wolf-Dietrich Rüdiger
(86) Internationale Anmeldenummer: PCT/EP2018/060601
(87) Internationale Veröffentlichungsnummer: WO 2018/197564

(56) Entgegenhaltungen:
- WO-A1-2009/073232
- WO-A1-2010/132783
- DE-T2- 69 931 584
- JP-A- H09 222 427
- US-A- 5 632 905
- US-B1- 6 280 400

## Beschreibung

Die Erfindung betrifft einen Trennkörper zum Trennen einer ersten von einer zweiten Phase einer Flüssigkeit in einem rohrförmigen Behälter. Gemeint sind insbesondere Trennkörper zum Trennen von Blutserum als erster Phase von Blutkuchen als zweiter Phase bei Blut als Flüssigkeit innerhalb eines Blutentnahmeröhrchens. Die Erfindung betrifft darüber hinaus auch den rohrförmigen Behälter.

Im Stand der Technik sind Blutentnahmeröhrchen mit Trennkörpern grundsätzlich bekannt. Im Auslieferungszustand der Blutentnahmeröhrchen sind die Trennkörper in einer Ausgangsposition fixiert. Wenn Blut über einen Zulauf in das Blutentnahmeröhrchen hineinfließt, wird der Trennkörper von dem Blut umströmt oder durchströmt; in der Ausgangsposition stellt der Trennkörper jedenfalls keine Dichtung für das Blut innerhalb des Blutentnahmeröhrchens dar. Zur medizinischen Analyse ist es erforderlich, dass das Blut in zwei Bestandteile, nämlich Blutserum und Blutkuchen aufgetrennt wird. Zu diesem Zweck wird das Blutentnahmeröhrchen mit dem darin befindlichen Blut zentrifugiert. Der schwerere Blutkuchen setzt sich dann aufgrund der Zentrifugation in dem bodennahen Volumenbereich des Blutentnahmeröhrchens ab, während das leichtere Blutserum auf dem Blutkuchen aufschwimmt. Unter Einwirkung der Zentrifugalkraft löst sich der Trennkörper aus seiner Ausgangsposition und wandert in eine Abdichtposition. Weil die Dichte des gesamten Trennkörpers in einem Wertebereich zwischen der Dichte des Blutserums und der Dichte des Blutkuchens liegt, positioniert sich der Trennkörper automatisch genau an der Phasengrenze zwischen Blutserum und Blutkuchen. Diese Position wird auch Abdichtposition genannt, weil der Trennkörper in dieser Position mit seinem Dichtrand umlaufend dichtend an der Innenseite des rohrförmigen Proberöhrchens anliegt und somit das Blutserum von dem Blutkuchen trennt. Der Trennkörper hält diese Abdichtposition auch nach Ende des Zentrifugierens bei, so dass das Blutserum und der Blutkuchen für eine Laboruntersuchung getrennt zur Verfügung stehen. Trennkörper sind offenbart, z. B. in der internationalen Patentanmeldung WO 2010/132783 A1. Die dort beschriebenen Trennkörper weisen jeweils einen aus elastischem Material gefertigten Schwimmkörper mit einem in der Draufsicht kreisförmigen umlaufenden Dichtrand auf, wobei der Dichtrand zum dichtenden Anliegen an der Innenseite eines rohrförmigen Probenbehälters in einer Abdichtposition ausgebildet ist. An der Unterseite des Schwimmkörpers ist jeweils ein Ballastkörper befestigt. Die Dichte des Ballastkörpers ist jeweils größer als die Dichte des Schwimmkörpers und die Dichte des gesamten Trennkörpers liegt in einem Wertebereich zwischen der Dichte der ersten Phase und der Dichte der zweiten Phase der Flüssigkeit.

Je nach Ausgestaltung der Trennkörper im Stand der Technik kann es beim Wandern des Trennkörpers in die Abdichtposition - aber bevor er diese erreicht hat - nachteiligerweise passieren, dass geringe Mengen der zweiten Phase der Flüssigkeit in den Bereich oberhalb des Trennkörpers in dem rohrförmigen Behälter gelangen und sich dort mit der ersten Phase der Flüssigkeit vermischen.

Aus dem Stand der Technik in Form der Druckschrift WO 2016/076911 A1 ist eine Trenneinheit für die Trennung einer Flüssigkeit in eine erste leichte und in eine zweite schwerere Phase unter Anwendung von Zentrifugalkraft bekannt, wobei die Flüssigkeit Blut sein kann. Ein rohrförmiger Behälter weist einen Trennkörper auf, wobei der Trennkörper im oberen Bereich einen Schwimmkörper und im unteren Bereich einen Ballastkörper aufweist. Der Trennkörper ist ausgebildet zum dichtenden Anliegen an der Innenseite des rohrförmigen Behälters. Die Dichte des Ballastkörpers ist dabei größer als die Dichte des Schwimmkörpers und die Dichte des Trennkörpers liegt zwischen der Dichte der ersten Phase und der Dichte der zweiten Phase der zu trennenden Flüssigkeit.

In der Druckschrift DE 699 31 584 T2 wird eine Vorrichtung zum Trennen einer Fluidprobe unter Zentrifugalkraft in eine Phase mit höherem spezifischen Gewicht und eine Phase mit niedrigerem spezifischen Gewicht beschrieben, wobei die Fluidprobe eine Blutprobe sein kann. Die Vorrichtung weist ein Separatorelement (Trennkörper) auf, das in einem zylindrischen Röhrchen angeordnet ist. Das Separatorelement weist einen Schwimmer im oberen Bereich und ein Ballastelement im unteren Bereich auf sowie einen Dichtungskörper zum dichtenden Anliegen an der Innenseite des Röhrchens. Die Dichte des Ballastelements ist dabei größer als die Dichte des Schwimmers und die Gesamtdichte des Separatorelements liegt zwischen der Dichte der ersten Phase und der Dichte der zweiten Phase der zu trennenden Flüssigkeit.

Die Druckschrift DE 600 23 823 T2 beinhaltet eine Vorrichtung zur Trennung einer flüssigen Probe (z. B. Blut) in eine erste Phase hoher Dichte und in eine Phase niedriger Dichte unter Einwirkung von Zentrifugalkraft. In einem Röhrchen mit zylindrischer Seitenwand ist ein Separator (Trennkörper) angeordnet, der im oberen Bereich einen Schwimmer und im unteren Bereich einen Ballastteil aufweist sowie einen Faltenbalg zum dichtenden Anliegen an der Innenseite des Röhrchens. Die Dichte des Ballastteils ist dabei größer als die Dichte des Schwimmers und die Gesamtdichte des Separators liegt zwischen den Dichten der ersten Phase und der zweiten Phase der zu trennenden Flüssigkeit.

Die Druckschriften US 5,632,905 A und US 6,280,400 B1 betreffen die Trennung einer Blutprobe in eine leichtere und eine schwerere Phase durch Zentrifugieren in einem Röhrchen. In dem Röhrchen ist ein Trennkörper angeordnet. Der Trennkörper hat in der US '905 eine scheibenförmige Form und in der US '400 B1 eine zylinderförmige Form. In beiden Druckschriften liegen die Trennkörper in einer Abdichtposition an der Phasengrenze zwischen leichterer und schwererer Phase.

Ausgehend von dem zitierten Stand der Technik liegt der Erfindung die Aufgabe zugrunde, einen bekannten Trennkörper zum Trennen einer ersten von einer zweiten Phase von Blut sowie einen bekannten rohrförmigen Behälter mit dem Trennkörper dahingehend weiterzubilden, dass der Dichtrand des Trennkörpers in einem vorgegebenen Abstand d größer Null oberhalb des Schwerpunkts des gesamten Trennkörpers liegt und dass der Abstand d zwischen der tiefsten Stelle des umlaufenden Dichtrands und dem Gesamtschwerpunkt des Trennkörpers einerseits nicht zu klein, aber andererseits auch nicht zu groß gewählt ist.

Diese Aufgabe wird im Hinblick auf den Trennkörper durch den Gegenstand des Anspruchs 1 gelöst.

Allgemein gilt: Die Dichte der zweiten Phase der Flüssigkeit ist größer als die Dichte der ersten Phase der Flüssigkeit. Für Blut als Flüssigkeit bedeutet dies, dass der Blutkuchen als zweite Phase eine größere Dichte aufweist als das Blutserum, welches der ersten Phase entspricht. Nach einer Zentrifugation schwimmt deshalb das Blutserum auf dem Blutkuchen auf. Die Dichte des gesamten Trennkörpers liegt in einem Wertebereich zwischen der Dichte der ersten Phase und der Dichte der zweiten Phase der Flüssigkeit. Deshalb positioniert sich der Trennkörper in der Abdichtposition immer auf der Phasengrenze zwischen den beiden Phasen.

Sofern nichts anderes gesagt ist, wird der Trennkörper nachfolgend in einer Normalposition beschrieben. In dieser Normalposition ist der Ballastkörper unterhalb des Schwimmkörpers angeordnet. Der Schwerpunkt des Schwimmkörpers, der Schwerpunkt des Ballastkörpers und der Schwerpunkt des gesamten Trennkörpers liegen alle auf einer vertikalen Linie, wobei der Schwerpunkt des gesamten Trennkörpers zwischen den Schwerpunkten des Schwimmkörpers und des Ballastkörpers liegt. Die nachfolgend verwendeten Begriffe wie vertikal, horizontal, unterhalb, oberhalb, Seitenansicht und Draufsicht etc. beziehen sich alle auf diese Normalposition. Die Abdichtposition entspricht der Normalposition bei senkrecht stehendem rohrförmigen Behälter.

Im Auslieferungszustand bzw. in seiner Ausgangsposition ist der Trennkörper in dem rohrförmigen Behälter lösbar eingeklemmt. Unter Einwirkung einer Kraft, insbesondere der Zentrifugalkraft, löst sich der Trennkörper aus dieser Ausgangsposition und wandert in die oben beschriebene Abdichtposition. Dabei dreht er sich um 90°. Weil die Dichte des gesamten Trennkörpers, wie gesagt, zwischen der Dichte der ersten Phase und der Dichte der zweiten Phase der Flüssigkeit liegt, positioniert sich der Trennkörper in der Abdichtposition genau so, dass sein Gesamtschwerpunkt auf der Phasengrenze, d. h. beispielsweise zwischen Blutserum und Blutkuchen liegt.

Um zu verhindern, dass auch nur geringe Mengen der zweiten Phase der Flüssigkeit in den Bereich oberhalb des Trennkörpers und damit in die erste Phase der Flüssigkeit gelangen, kurz bevor der Trennkörper die Abdichtposition eingenommen hat, sieht die Erfindung vor, dass der Dichtrand des Schwimmkörpers in einem vorgegebenen Abstand größer Null oberhalb des Schwerpunktes des gesamten Trennkörpers ausgebildet ist. Je größer dieser Abstand ist, desto sicherer wird eine Vermischung der ersten mit der zweiten Phase oberhalb des Trennkörpers verhindert. Dies ist wichtig für die medizinische Analyse von insbesondere der ersten Phase der Flüssigkeit, d. h. des Blutserums.

Gemäß Anspruch 1 liegt der Abstand in einem Intervall von 0,05 mm bis 4 mm, gemäß Anspruch 2 vorzugsweise jedoch in einem Intervall von 1 mm bis 3 mm. Der gewählte Abstand aus diesem Intervall stellt einen Kompromiss dar. Wie gesagt, einerseits muss er eine gewisse Mindestgröße haben, um die sonst drohende Vermischung von erster und zweiter Phase sicher zu verhindern; andererseits sollte er auch nicht zu groß gewählt werden, weil ansonsten eine zu große Menge der ersten Phase der Flüssigkeit verlorengeht und nicht mehr für die medizinische Analyse zur Verfügung steht. Bei der verlorengegangenen Menge an erster Phase handelt es sich um diejenige Restmenge, welche sich nach Positionierung des Trennkörpers in der Abdichtposition unterhalb des Dichtrandes des Trennkörpers und oberhalb der Phasengrenze befindet bzw. einstellt.

Je nach Ausgestaltung des Trennkörpers und insbesondere von dessen Schwimmkörper kann der Dichtrand - in einer Seitenansicht betrachtet - in Umfangsrichtung gerade und horizontal verlaufend oder aber wellenförmig verlaufend ausgebildet sein. In letzterem Fall bemisst sich der Abstand von dem tiefsten Wellental zu dem Schwerpunkt des gesamten Trennkörpers.

Die technische Lehre der vorliegenden Erfindung gilt für beliebig gestaltete Trennkörper. Insbesondere gilt sie sowohl für Trennkörper, deren Schwimmkörper scheibenförmig, mit sphärischer Verformung oder ohne sphärische Verformung ausgebildet sind, wie auch für kugelförmige oder topfförmige Trennkörper. Gleichermaßen gilt sie auch für Trennkörper mit beliebig gestalteten Ballastkörpern. So gilt sie insbesondere auch für Ballastkörper, welche in Form einer Mehrzahl von Fingern ausgebildet sind, die sich von der Unterseite eines scheibenförmigen Schwimmkörpers weg erstrecken.

Die oben genannte Aufgabe der Erfindung wird bezüglich des rohrförmigen Behälters durch den Gegenstand des Anspruchs 6 gelöst. Die Vorteile dieser Lösung entsprechen den oben mit Bezug auf den Trennkörper genannten Vorteilen.

### Der Beschreibung sind sieben Figuren beigefügt, wobei

- Figur 1: einen Trennkörper gemäß der Erfindung mit scheibenförmigem Schwimmkörper gemäß einem ersten Ausführungsbeispiel;
- Figur 2: den Trennkörper nach Figuren 1 in einem rohrförmigen Behälter;
- Figur 3: eine vergrößerte Detaildarstellung von Figur 2;
- Figur 4: einen Trennkörper gemäß einem zweiten Ausführungsbeispiel;
- Figur 5: einen Trennkörper gemäß einem dritten Ausführungsbeispiel;
- Figur 6: einen Trennkörper gemäß einem vierten Ausführungsbeispiel; und
- Figur 7: einen Trennkörper gemäß einem fünften Ausführungsbeispiel zeigt.

Die Erfindung wird nachfolgend unter Bezugnahme auf die genannten Figuren in Form von Ausführungsbeispielen detailliert beschreiben. In allen Figuren sind gleiche technische Elemente mit gleichen Bezugszeichen bezeichnet.

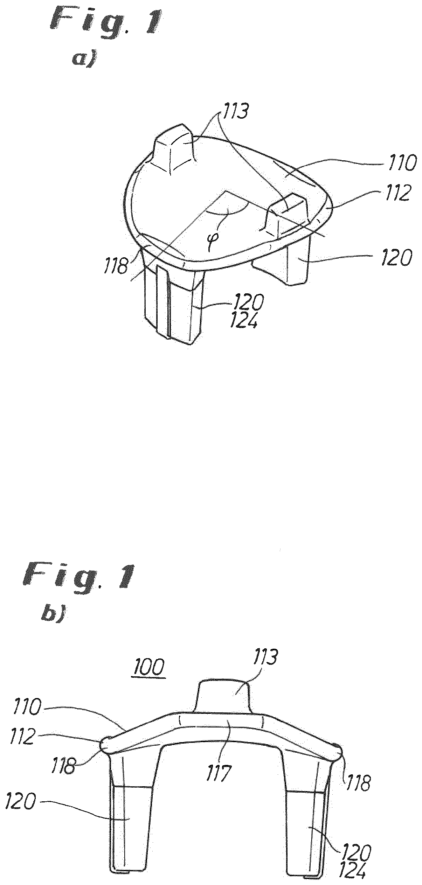

Figuren 1 a + b zeigen einen erfindungsgemäßen Trennkörper 100 gemäß einem ersten Ausführungsbeispiel in perspektivischer Ansicht und in einer Seitenansicht. Sein Schwimmkörper 110 ist grundsätzlich scheibenförmig ausgebildet, allerdings ist er sphärisch verformt. Deshalb ist der umlaufende Rand 112 von der Seite betrachtet wellenförmig mit Wellentälern 118 und Wellenbergen 117 ausgebildet. Von der Unterseite des scheibenförmigen Schwimmkörpers 110 stehen jeweils im Bereich der Wellentäler 118 Ballastkörper 120 in Form von Fingern 124 nach unten ab. Im Bereich der Wellenberge 117 sind auf der Oberseite des scheibenförmigen Schwimmkörpers Materialansammlungen vorzugsweise aus dem Material des Schwimmkörpers ausgebildet; diese wirken als zusätzliche Auftriebskörper 113. Die Finger 124 und die Auftriebskörper 113 sind bei dem in den Figuren 1 a + b gezeigten Ausführungsbeispiel jeweils mit einem Umfangswinkelabstand von ϕ=90° an der Peripherie des scheibenförmigen Schwimmkörpers abwechselnd verteilt angeordnet.

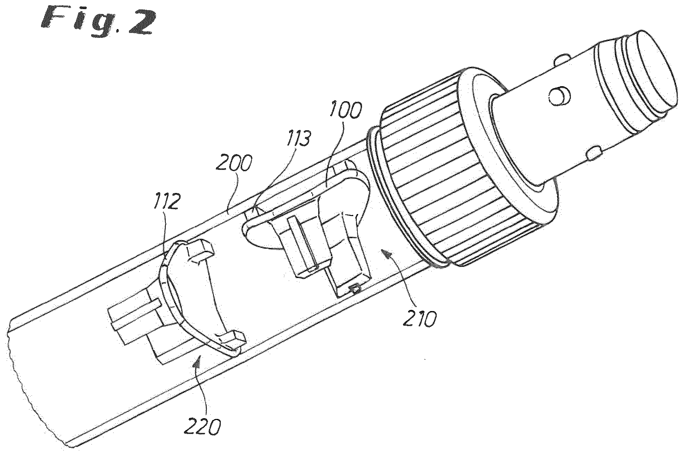

Figur 2 zeigt den Trennkörper gemäß den Figuren 1 a + b im Inneren des rohrförmigen Behälters 200. Im Auslieferungszustand befindet sich der Trennkörper in seiner Ausgangsposition 210. Er stützt sich dann einerseits mit den freien Enden der Finger 124 und andererseits mit den freien Enden der Auftriebskörper 113 an der Innenseite des rohrförmigen Behälters 200 ab. Die freien Enden der Auftriebskörper 113 sind deshalb vorzugsweise entsprechend dem Innenradius des rohrförmigen Behälters 200 abgerundet. In der Ausgangsposition 210 kann der Trennkörper 100 von in den rohrförmigen Behälter einfließendem Blut umströmt werden, sodass das Blut auch in tiefere Volumenbereiche des rohrförmigen Behälters gelangen kann.

Unter Einwirkung einer Kraft, insbesondere der Zentrifugalkraft löst sich der Trennkörper 100 aus seiner Ausgangsposition 210 und wandert in eine Abdichtposition 220. Dabei dreht er sich um 90°. Erst wenn der Trennkörper 100 nicht mehr unter der Einwirkung der Zentrifugalkraft steht, verformt er sich wieder zurück in seinen Ausgangszustand. Sein Dichtrand 112 liegt dann in der Abdichtposition 220 in Umfangsrichtung R überall dichtend an der Innenseite des rohrförmigen Behälters 200 an und separiert auf diese Weise die beiden Phasen der Flüssigkeit bzw. des Blutes wirksam voneinander.

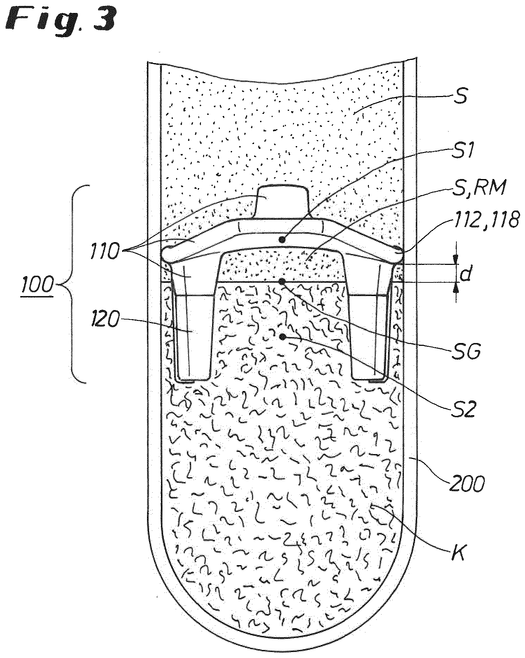

Figur 3 zeigt einen vergrößerten Ausschnitt aus der Figur 2. Konkret zeigt Figur 3 den Trennkörper 100 in der Abdichtposition innerhalb des rohrförmigen Behälters 200. Das Bezugszeichen S1 bezeichnet den Schwerpunkt des Schwimmkörpers 110; das Bezugszeichen S2 bezeichnet den Schwerpunkt des Ballastkörpers 120. Der Schwerpunkt des gesamten Trennkörpers 100 ist mit dem Bezugszeichen SG bezeichnet.

Figur 3 zeigt den senkrecht stehenden Behälter 200 mit darin befindlicher Flüssigkeit nach erfolgter Zentrifugation. Aufgrund der Zentrifugation hat sich die Flüssigkeit, beispielsweise Blut, in zwei Phasen separiert. Die Dichte der ersten Phase, beispielsweise Blutserum S ist geringer als die Dichte der zweiten Phase, bei beispielsweise Blutkuchen K. Deshalb schwimmt die erste Phase mit der geringeren Dichte nach erfolgter Zentrifugation auf der zweiten schwereren Phase auf. Dies ist in Figur 3 gezeigt. Dort schwimmt das leichtere Blutserum auf dem schwereren Blutkuchen auf.

Die Dichte des gesamten Trennkörpers 100 liegt in einem Wertebereich zwischen der Dichte der ersten Phase und der Dichte der zweiten Phase der Flüssigkeit bzw. des Blutes. Deshalb positioniert sich der Trennkörper 100 in Figur 3 in der gezeigten Abdichtposition automatisch so, dass sein Gesamtschwerpunkt SG genau auf der Phasengrenze zwischen der ersten und der zweiten Phase der Flüssigkeit liegt.

Wie oben erläutert, sieht die vorliegende Erfindung vor, dass der Dichtrand 112 des Trennkörpers bzw. des Schwimmkörpers 110 einen vorgegebenen Abstand d zu dem Schwerpunkt SG des gesamten Trennkörpers aufweist. Dieser Abstand d, auch Sicherheitsabstand genannt, sollte, wie oben erläutert, einerseits nicht zu groß, andererseits jedoch auch nicht zu klein gewählt werden. Wird er zu klein gewählt, besteht die Gefahr, dass die erste Phase der Flüssigkeit oberhalb des Trennkörpers durch Anteile der zweiten Phase K verunreinigt wird, während sich der Trennkörper 100 in die Abdichtposition begibt. Wenn er andererseits zu groß gewählt wird, geht eine zu große Menge an der ersten Phase der Flüssigkeit verloren; das ist diejenige Restmenge RM an der ersten Phase der Flüssigkeit, welche sich zwischen dem Dichtrand 112 des Trennkörpers in der Abdichtposition und der Phasengrenze ansammelt. Diese Restmenge RM steht für die medizinische Analyse der ersten Phase der Flüssigkeit nicht mehr zur Verfügung. Erfindungsgemäß liegt dieser Abstand d beispielsweise in einem Intervall von 0,05 mm bis 4 mm, vorzugsweise zwischen 1 mm bis 3 mm. Der Abstand d bemisst sich grundsätzlich immer von der Unterkante des Dichtrandes 112 bis zur Phasengrenze. Bei wellenförmigem Dichtrand, wie in Figur 3 gezeigt, bemisst er sich von dem tiefsten Wellental 118 des Dichtrandes bis zu dem Schwerpunkt SG des gesamten Trennkörpers bzw. bis zur Phasengrenze.

Wichtig ist, dass die soeben beschriebene technische Lehre der Erfindung für beliebige Trennkörper gilt. Sie gilt insbesondere nicht nur für den Trennkörper gemäß der erfindungsgemäßen ersten Ausführungsform, wie er in den Figuren 1 bis 3 gezeigt ist.

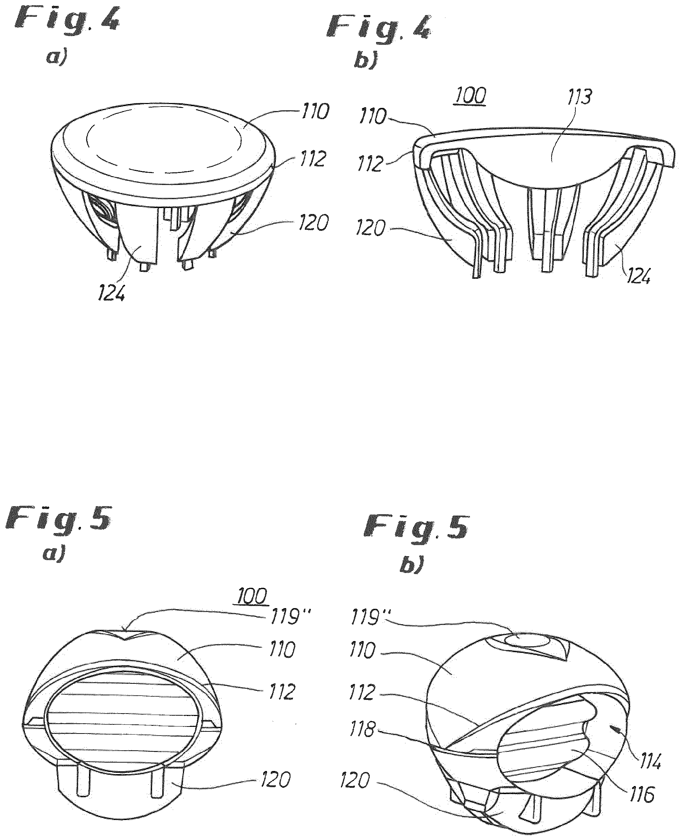

In den nachfolgenden Figuren 4 bis 7 sind weitere mögliche Trennkörper gezeigt; diese werden nachfolgend kurz beschrieben; allerdings ist, wie gesagt, die technische Lehre der Erfindung auch nicht auf diese Ausführungsformen der Trennkörper beschränkt.

Figur 4 zeigt den erfindungsgemäßen Trennkörper 100 gemäß einem zweiten Ausführungsbeispiel in einer perspektivischen Ansicht und in einer Querschnittsdarstellung. Der Trennkörper 100 besteht aus einem Schwimmkörper 110 und einem Ballastkörper 120. Der Schwimmkörper 110 ist scheibenförmig mit einer Verdickung als Auftriebskörper 113 in seiner Mitte und mit einem umlaufenden Dichtrand 112 ausgebildet. Der Ballastkörper 120 ist in Form einer Mehrzahl von Fingern 124 ausgebildet, welche sich von der Unterseite des scheibenförmigen Schwimmkörpers 110 weg erstrecken. Die Finger 124 sind an dem Rand des Ballastkörpers verteilt angeordnet.

Figur 5 zeigt den erfindungsgemäßen Trennkörper 100 in kugelförmiger Ausgestaltung (3. Ausführungsbeispiel). Er besteht aus dem elastischen Schwimmkörper 110 mit dem in der Draufsicht kreisförmigen Dichtrand 112. Dem steht nicht entgegen, dass der Dichtrand 112 in der Seitenansicht nach Figur 5 wellenförmig ausgebildet ist. Das Bezugszeichen 118 bezeichnet ein Wellental des Dichtrandes 112. Der Abstand d bemisst sich von diesem Wellental abwärts bis zum Gesamtschwerpunkt des Trennkörpers.

An der Unterseite des Schwimmkörpers 110 ist ein Ballastkörper 120 befestigt. Gemäß Figur 5 ist der Schwimmkörper 110 lokal verengt. Im Bereich der Verengung 114 ist er als Membran 116 ausgebildet. Die Membran ist wellenförmig ausgebildet mit Wellenbergen und Wellentälern. Unabhängig davon oder alternativ könnte die Membran 116 auch aus elastischem Material gebildet sein. Die Ausbildung der Membran in Wellenform und / oder aus elastischem Material ist erforderlich, um eine Federwirkung der Membran zu ermöglichen. Die Membran wirkt als Zugfeder, wodurch der Schwimmkörper und der Ballastkörper ein stückweit näher aufeinander zu bewegt werden. Dadurch verbreitert sich der Trennkörper, insbesondere der Schwimmkörper, mit der Folge, dass der Dichtrand in der Abdichtposition mit einem ausreichend großen Druck gegen die Innenseite des Behälters drückt, um die beiden Phasen der Flüssigkeit wirksam gegeneinander abzugrenzen.

An seiner dem Ballastkörper 120 abgewandten Oberseite weist der Schwimmkörper 110 eine lokale Abflachung 119" bzw. Sicke auf. Im Bereich der Abflachung liegt der Schwimmkörper in der Ausgangsposition nicht dichtend an der Innenseite des Behälters an und ermöglicht so ein dortiges Umströmen des Trennkörpers mit der in den Behälter einströmenden Flüssigkeit.

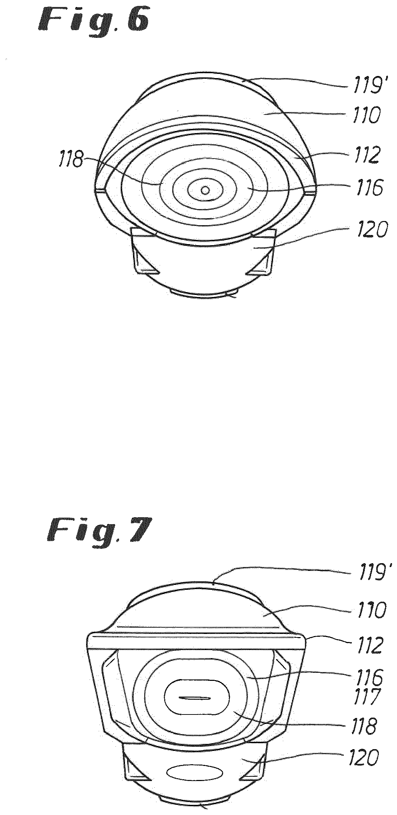

Figur 6 zeigt den kugelförmigen Trennkörper 100 in einer vierten Ausführungsform. Diese Ausführungsform unterscheidet sich im Wesentlichen nur in der Form der Membran 116 und in der Gestaltung der Oberfläche des Schwimmkörpers von der Ausführungsform gemäß Fig. 5.

Die Wellenberge und die Wellentäler der Membran 116 sind zwar auch hier ringförmig, allerdings sind sie hier oval ausgebildet. Anstelle der Abflachung weist die Oberseite des Schwimmkörpers 110 eine Erhebung 119' auf. Die Abflachung und die Erhebung bewirken gleichermaßen, dass sich in ihren Umgebungen keine Reste der Flüssigkeit, insbesondere keine Blutreste ansammeln können, die zwischen dem Trennkörper und der Wand des Behälters nicht abließen können.

Figur 7 zeigt den Trennkörper 100 in einer fünften topfförmigen Ausführung. Abgesehen von seiner äußeren (Topf-) Form unterscheidet sich dieser Trennkörper im Wesentlichen nur in dem umlaufenden Dichtrand 112 von dem Trennkörper gemäß der Figuren 5 und 6. Der Dichtrand 112 ist hier nicht wellenförmig, sondern grade horizontal verlaufend ausgebildet.

### Bezugszeichenliste

- 100: Trennkörper
- 110: Schwimmkörper
- 112: Dichtrand
- 113: Auftriebskörper/Materialverdickung
- 116: Membran
- 117: Wellenberg
- 118: Wellentäler
- 119': Erhebung
- 119": Abflachung/ Sicke
- 120: Ballastkörper
- 124: Finger
- 200: Behälter
- 210: Ausgangsposition
- 220: Abdichtposition

- d: Abstand
- K: Blutkuchen, allg. zweite Phase der Flüssigkeit
- R: Umfangsrichtung
- S: Blutserum, allg. erste Phase der Flüssigkeit
- S1: Schwerpunkt des Schwimmkörpers
- S2: Schwerpunkt des Ballastkörpers
- SG: Schwerpunkt des gesamten Trennkörpers
- RM: Restmenge
- ϕ: Umfangswinkelabstand

## Patentansprüche

1. Trennkörper (100) zum Trennen einer ersten Phase in Form von Blutserum (S) von einer zweiten Phase in Form von Blutkuchen (K) bei Blut als Flüssigkeit, unter Zentrifugalkraft in einem rohrförmigen Behälter (200); aufweisend:
einen aus elastischem Material gefertigten Schwimmkörper (110) mit einem in der Draufsicht umlaufenden Dichtrand (112) zum dichtenden Anliegen an der Innenseite des rohrförmigen Behälters (200) in einer Abdichtposition (220); und
mindestens einen an der Unterseite des Schwimmkörpers (110) befestigten Ballastkörper (120);
wobei die Dichte des Materials des Ballastkörpers (120) größer ist als die Dichte des Materials des Schwimmkörpers (110);
wobei die Dichte des Trennkörpers (100) in einem Wertebereich zwischen der Dichte der ersten Phase und der Dichte der zweiten Phase der Flüssigkeit liegt, weshalb der Schwerpunkt des Trennkörpers genau auf der Phasengrenze zwischen der ersten und der zweiten Phase der Flüssigkeit liegt;
wobei der Dichtrand (112) des Schwimmkörpers (110) in einem Abstand (d) oberhalb des Schwerpunktes (SG) des Trennkörpers (100) ausgebildet ist; und
wobei der Dichtrand (112) - in der Seitenansicht betrachtet - in Umfangsrichtung (R) horizontal verlaufend ausgebildet ist, oder
wobei der Dichtrand (112) - in der Seitenansicht betrachtet - in Umfangsrichtung (R) unter Ausbildung von Wellenbergen (117) und Wellentälern (118) wellenförmig verlaufend ausgebildet ist; und sich der Abstand (d) in diesem Fall von dem tiefsten Wellental (118) zu dem Schwerpunkt (SG) des gesamten Trennkörpers (100) bemisst;
**dadurch gekennzeichnet,**
**dass** der Abstand (d) in einem Intervall von 0,05 mm bis 4 mm liegt.

2. Trennkörper (100) nach Anspruch 1,
**dadurch gekennzeichnet,**
**dass** der Abstand (d) in einem Intervall von 1 mm - 3 mm liegt.

3. Trennkörper (100) nach Anspruch 1 oder 2,
**dadurch gekennzeichnet, dass**
der Schwimmkörper (110) scheibenförmig, beispielsweise mit sphärischer Verformung ausgebildet ist.

4. Trennkörper (100) nach Anspruch 3,
**dadurch gekennzeichnet,**
**dass** der Ballastkörper (120) in Form einer Mehrzahl von Fingern (124) ausgebildet ist, welche sich von der Unterseite des scheibenförmigen Schwimmkörpers (110) - an dessen Rand verteilt - weg erstrecken.

5. Trennkörper (100) nach Anspruch 1 oder 2,
**dadurch gekennzeichnet, dass**
der Schwimmkörper (110) kugelförmig oder topfförmig ausgebildet ist.

6. Rohrförmiger Behälter mit einem Trennkörper nach einem der vorangegangenen Ansprüche, wobei der Trennkörper innerhalb des Behälters insbesondere eine Abdichtposition einnehmen kann.

## Claims

1. Separator (100) for separating a first phase in the form of blood serum (S), from a second phase in the form of clotted blood (K), in the case of blood as fluid, under centrifugal force in a tube-shaped container (200); comprising
a floating element (110) made of an elastic material, with, when viewed from above, a circumferential sealing edge (112) for application in a sealing manner on the inner side of the tube-shaped container (200) in a sealing position (220); and
at least one ballast element (120) fastened on the underside of the floating element (110);
wherein the density of the material of the ballast element (120) is greater than the density of the material of the floating element (110);
wherein the density of the separator (100) is within a value range between the density of the first phase and the density of the second phase of the fluid, for which reason the centre of gravity of the separator lies precisely on the phase boundary between the first and the second phase of the fluid;
wherein the sealing edge (112) of the floating element (110) is located at a distance (d) above the centre of gravity (SG) of the separator (100); and
wherein the sealing edge (112) - seen from the side - is configured to extend horizontally in the circumferential direction (R), or
wherein the sealing edge (112) - seen from the side - is configured to extend in the circumferential direction (R) in an undulating manner forming wave peaks (117) and wave troughs (118); and, in this case, the distance (d) is measured from the lowest wave trough (118) to the centre of gravity (SG) of the entire separator (100);
**characterised in that**
the distance (d) is within a range of 0.05 mm to 4 mm.

2. Separator (100) according to claim 1
**characterised in that**
the distance (d) is within a range of 1 mm - 3 mm.

3. Separator (100) according to claim 1 or 2
**characterised in that**
the floating element (110) is designed to be disk-shaped, for example with spherical deformation.

4. Separator (100) according to claim 3
**characterised in that**
the ballast element (120) is designed in the form of a plurality of fingers (124) which extend away from the underside of the disk-shaped floating element (110) - distributed on its edge.

5. Separator (100) according to claim 1 or 2
**characterised in that**
the floating element (110) is spherical or pot-shaped in design.

6. Tube-shaped container with a separator according to any one of the preceding claims, wherein the separator can, in particular, assume a sealing position within the container.

## Revendications

1. Séparateur (100) pour séparer une première phase sous forme de sérum sanguin (S) d'une seconde phase sous forme de sang coagulé (K) dans du sang en tant que liquide, par force centrifuge dans un contenant tubulaire (200) ; présentant :
un flotteur (110) fabriqué en matériau élastique comprenant un bord d'étanchéité (112) périphérique en vue de dessus pour le placement en étanchéité contre l'intérieur du contenant tubulaire (200) dans une position d'étanchéité (220) ; et
au moins un corps de ballast (120) fixé sur la sous-face du flotteur (110) ;
dans lequel la densité du matériau du corps de ballast (120) est supérieure à la densité du matériau du flotteur (110) ;
dans lequel la densité du séparateur (100) repose dans une plage de valeurs entre la densité de la première phase et la densité de la seconde phase du liquide, ce pourquoi le centre de gravité du séparateur repose précisément sur la limite de phase entre les première et seconde phases du liquide ;
dans lequel le bord d'étanchéité (112) du flotteur (110) est formé à un écart (d) au-dessus du centre de gravité (SG) du séparateur (100) ; et
dans lequel le bord d'étanchéité (112), en vue latérale, est conçu avec un tracé horizontal dans le sens périphérique (R), ou
dans lequel le bord d'étanchéité (112), en vue latérale, est conçu avec un tracé ondulé dans le sens périphérique (R), par la formation de crêtes (117) et de creux (118) ; et l'écart (d) se mesure dans ce cas du creux (118) le plus profond jusqu'au centre de gravité (SG) de la totalité du séparateur (100) ;
**caractérisé en ce que**
l'écart (d) repose dans un intervalle de 0,05 mm à 4 mm.

2. Séparateur (100) selon la revendication 1,
**caractérisé en ce que**
l'écart (d) repose dans un intervalle de 1 mm à 3 mm.

3. Séparateur (100) selon la revendication 1 ou 2,
**caractérisé en ce que**
le flotteur (110) est conçu en forme de disque, par exemple avec une déformation sphérique.

4. Séparateur (100) selon la revendication 3,
**caractérisé en ce que**
le corps de ballast (120) est conçu sous forme d'une pluralité de doigts (124) qui s'étendent en s'éloignant de la sous-face du flotteur (110) en forme de disque, répartis sur son bord.

5. Séparateur (100) selon la revendication 1 ou 2,
**caractérisé en ce que**
le flotteur (110) est en forme de sphère ou de cuvette.

6. Contenant tubulaire comprenant un séparateur selon l'une des revendications précédentes, dans lequel le séparateur peut adopter en particulier une position d'étanchéité à l'intérieur du contenant.
